# EUROPÄISCHE PATENTSCHRIFT

(11) **EP 0 514 893 B1**
(45) Veröffentlichungstag und Bekanntmachung des Hinweises auf die Patenterteilung: **26.01.1994**
(21) Anmeldenummer: 92108595.7
(22) Anmeldetag: 21.05.1992
(51) Int. Cl.: C07C 67/343, C07C 69/716

(54) **Verfahren zur Herstellung von beta-Ketocarbonsäureestern**
Process for the preparation of esters of beta-ketocarboxylic acids
Procédé de préparation d'esters d'acides bêta-cétocarboxyliques

(30) Priorität: 23.05.1991 DE 4116906
(43) Veröffentlichungstag der Anmeldung: 25.11.1992
(73) Patentinhaber: WACKER-CHEMIE GMBH, D-81737 München (DE)
(72) Erfinder: Meier, Josef, Dr., W-8263 Burghausen (DE)

(56) Entgegenhaltungen:
- JP-A-57 070 837
- JP-A-62 228 041
- HOUBEN-WEYL, METHODEN DER ORGANISCHEN CHEMIE, 4. Auflage, 1952, Band VIII, S. 615-616

## Beschreibung

Die Erfindung betrifft ein Verfahren zur Herstellung von β-Ketocarbonsäureestern ausgehend von Acetcarbonsäureestern und Carbonsäurechloriden.

Für die Herstellung von β-Ketoestern sind eine Reihe von Verfahren bekannt. So werden zum Beispiel Carbonsäureester als Carbonylkomponente mit CH-aciden Carbonsäureestern zu β-Ketocarbonsäureestern unter Verwendung von mindestens äquimolaren Mengen an Basen wie zum Beispiel Natriumhydrid, Natriumamid und Alkalimetallalkoholaten in einem inerten Lösungsmittel umgesetzt. Gemischte Esterkondensationen werden dabei im allgemeinen nur mit Ameisensäureestern als Carbonylkomponente durchgeführt, da andernfalls Reaktionsgemische entstehen. Die hierbei erzielten Ausbeuten sind jedoch relativ niedrig, da der bei der Kondensationsreaktion entstehende β-Ketoester eine höhere Reaktivität aufweist als die umzusetzenden Ausgangsverbindungen, was zu zahlreichen Nebenreaktionen führen kann. Derartige Herstellungsverfahren sind in den US-Patentschriften US-A 2407942 und US-A 2367632 beschrieben.

Weiterhin wird in der DE-A 2412784 die Kondensation von CH-aciden Dialkylketonen mit Dialkylcarbonaten mit mindestens äquimolaren Mengen Base zur Herstellung von β-Ketoestern beschrieben. Das Verfahren hat jedoch den Nachteil, daß das hochtoxische Hexamethylphosphorsäuretriamid (HMPT) als Lösungsmittel eingesetzt werden muß, um zu guten Ausbeuten zu gelangen.

In J.Am.Chem.Soc. 67 2198 (1945) wird beschrieben, Natriumacetessigester mit Carbonsäurechloriden zu acylieren und den entstandenen Acylacetessigester mit Ammoniak oder Natriummethylat zum β-Ketoester zu spalten. Es werden dabei jedoch nur Ausbeuten in der Größenordnung von 30 bis 40 % erreicht.

Aus der britischen Patentschrift GB-B 1000709 und aus Helv. 35, 2280 (1952) ist bekannt, β-Ketoester durch Umsetzung von Acetessigestern mit Carbonsäurechloriden in Gegenwart von Magnesiumalkoholaten und anschließender hydrolytischer Spaltung des 2-Acylacetessigesters herzustellen. Es treten jedoch auch bei dieser Methode in der Praxis Schwierigkeiten auf. So ist zum Beispiel die Aktivität von käuflichem Magnesiumalkoholat nicht ausreichend, sodaß das zur Umsetzung benötigte Magnesiumalkoholat stets frisch hergestellt werden muß. Dazu muß aber der toxikologisch sehr bedenkliche Tetrachlorkohlenstoff eingesetzt werden.

Aus der JP-A-5770837 ist ein Verfahren zur Herstellung von Magnesium- oder Calcium-Komplexen von β-Ketoestern bekannt, in welchem Acetessigsäurealkylester mit Oxiden oder Hydroxiden des Magnesiums oder Calciums umgesetzt werdenund anschließend mit 2.2-Dimethylpropionsäurehalogenid acyliert werden. Die JP-A-62228041 beschreibt am Beispiel eines Calcium-Chelatkomplexes von Acetessigester, welcher mit 2.2-Dimethylpropionsäurechlorid, analog der Verfahrensweise aus der JP-A-57070837 acyliert wurde, die Freisetzung des β-Ketoestesters (hier: 4.4-Dimethyl-3-oxo-valeriansäureester) mittels Umsetzung des Komplexes mit verdünnter Salzsäure. Nachteilig ist die dabei auftretende Nebenreaktion der Rückspaltung zum Acetessigester.

Es bestand somit die Aufgabe, ein Verfahren zur Herstellung von β-Ketoestern zur Verfügung zu stellen, das ausgehend von leicht zugänglichen Ausgangsprodukten, die β-Ketoester auf einfache und kostengünstige Weise in guten Ausbeuten liefert.

Es wurde gefunden, daß β-Ketoester in sehr guten Ausbeuten erhalten werden, wenn Acetcarbonsäureester mit Calciumhydroxid oder Calciumoxid in einem inerten Lösungsmittel umgesetzt wird, der gebildete Calciumkomplex mit Carbonsäurechlorid acyliert und anschließend mit einer Ammoniumsalzlösung unter Bildung von β-Ketoestern gespalten wird.

Gegenstand der Erfindung ist ein Verfahren zur Herstellung von β-Ketocarbonsäureestern der allgemeinen Formel 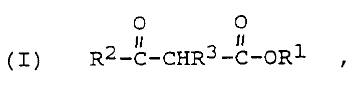
wobei R¹ für einen Alkylrest mit 1 bis 4 C-Atomen steht, R² einen Alkylrest oder Alkenylrest mit 2 bis 15 C-Atomen oder einen Phenylrest bedeutet und R³ Wasserstoff oder einen Alkyl- bzw. Alkenyl-Rest mit 1 bis 6 C-Atomen bedeutet, in welchem Acetcarbonsäureester der allgemeinen Formel 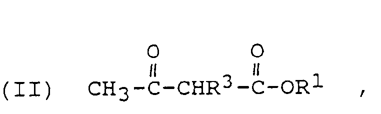
wobei R¹ und R³ die angegebenen Bedeutungen haben, mit Calciumhydroxid oder Calciumoxid in Gegenwart eines organischen Lösungsmittels und unter Ausschluß von Wasser umgesetzt werden, die gebildeten Calcium-Chelatkomplexe mit Carbonsäurechlorid acyliert werden, dadurch gekennzeichnet, daß die acylierten Calcium-Chelatkomplexe anschließend mit wäßriger Ammoniumsalzlösung unter Bildung der β-Ketocarbonsäureester der Formel (I) gespalten werden.

Bevorzugte Beispiele für die Reste R¹ sind der Methyl- oder Ethyl-Rest. Geeignete Reste R² sind geradkettige, verzweigte oder cyclische, gegebenenfalls substituierte, Alkylreste oder Alkenylreste wie Ethyl-, n-Propyl-, iso-Propyl-, n-Butyl-, iso-Butyl-, n-Pentyl-, iso-Pentyl-, Hexyl-, Heptyl-, Cyclohexylmethyl-, Undecenyl- oder Pentadecenylrest. Geeignete Reste R² sind weiterhin unsubstituierte oder substituierte Phenylreste. Beispiele für R³ sind Wasserstoff, Methyl-, Ethyl- oder n-Propyl-Rest. Vorzugsweise ist R³ Wasserstoff.

Zur Darstellung des Calcium-Chelatkomplexes werden die Acetcarbonsäureester der Formel (II) mit Calciumhydroxid oder Calciumoxid, vorzugsweise mit Calciumhydroxid, umgesetzt.

Die Calciumverbindungen werden dabei unter wasserfreien Bedingungen in einem organischen Lösungsmittel suspendiert, wobei durch mechanische Bewegung für eine gute Verteilung der Reaktionspartner gesorgt wird. Vorzugsweise werden aprotische Lösungsmittel eingesetzt. Beispiele hierfür sind Chlorkohlenwasserstoffe wie Dichlormethan, 1,1,1-Trichlorethan und Trichlorethylen. Es können auch aromatische Kohlenwasserstoffe wie Benzol oder Toluol, Ether wie Diethylether, Diisopropylether oder Tetrahydrofuran oder Ketone wie Isopropylmethylketon oder Isobutylmethylketon verwendet werden. Besonders bevorzugt werden Dichlormethan oder Gemische der genannten Lösungsmittel mit Dichlormethan.

Die Umsetzung erfolgt bei Temperaturen von 0 bis 50°C, vorzugsweise bei 20 bis 30°C, wobei das Reaktionsgemisch durch Kühlung auf Reaktionstemperatur gehalten wird. Die Calciumverbindung wird vorzugsweise in mindestens äquivalenter Menge zum Acetcarbonsäureester, besonders bevorzugt in einem Überschuß, insbesonders in einem Überschuß von bis zu 5 Mol%, eingesetzt.

Zur Einführung des Restes R² wird der in der Vorstufe hergestellte Calcium-Chelatkomplex mit dem entsprechenden Carbonsäurechlorid acyliert. Das Carbonsäurechlorid wird dabei vorzugsweise in mindestens äquivalenter Menge, besonders bevorzugt in einem Überschuß, insbesonders in einem Überschuß von 10 bis 20 Mol%, jeweils bezogen auf den Acetcarbonsäureester, der Suspension zugegeben. Die Reaktionstemperatur beträgt 0 bis 50°C, vorzugsweise 30 bis 40°C.

Zur Bildung der gewünschten β-Ketocarbonsäureester der allgemeinen Formel (I) wird die Suspension anschließend mit einer wäßrigen Lösung eines Ammoniumsalzes, vorzugsweise Ammoniumformiat, Ammoniumacetat oder Ammoniumchlorid, besonders bevorzugt mit einer wäßrigen Ammoniumchlorid-Lösung, versetzt. Die Ammoniumsalz-Konzentration in der Lösung beträgt vorzugsweise 10 bis 20 Gew%. Vorzugsweise wird das Ammoniumsalz in etwa äquimolarer Menge, bezogen auf den Acetcarbonsäureester, eingesetzt. Durch Zugabe von Base, vorzugsweise Ammoniak oder wasserlösliche primäre bzw. sekundäre Amine, besonders bevorzugt wäßrigem Ammoniak wird in der Suspension ein pH-Wert von 8.8 bis 9.5 eingestellt. Die Umsetzung erfolgt unter Bildung des β-Ketocarbonsäureesters bei Temperaturen im Bereich von 0 bis 50°C, vorzugsweise 30 bis 40°C. Nach Beendigung der Umsetzung kann der gebildete β-Ketocarbonsäureester mittels üblicher Techniken wie Extraktion oder Eindampfen isoliert werden und anschließend beispielsweise durch fraktionierte Destillation weiter gereinigt werden.

Die mit dem erfindungsgemäßen Verfahren leicht zugänglichen β-Ketocarbonsäureester können als wichtige Zwischenprodukte in der Synthese von pharmazeutischen Wirkstoffen oder Pflanzenschutzmitteln eingesetzt werden. Speziell Propionylessigester eignet sich auch als Grundstoff für biologisch abbaubare Polymere.

In den folgenden Beispielen wird das erfindungsgemäße Verfahren näher erläutert:

### Beispiel 1:

77.8 g (1.05 Mol) Calciumhydroxid wurden in 550 ml Methylenchlorid vorgelegt und unter kräftigem Rühren bei 20 bis 30°C innerhalb von 20 Minuten 116 g (1.0 Mol) Acetessigsäuremethylester zugetropft. Anschließend wurde eine halbe Stunde nachgerührt. Zu der dicken Suspension wurden dann innerhalb von 1.5 Stunden 122.6 g (1.15 Mol) Buttersäurechlorid bei einer Temperatur von 30 bis 35°C zudosiert.

Anschließend wurde 2 Stunden lang bei 40°C nachgerührt. Zur Reaktionsmischung wurden dann bei 30°C 56.2 g (1.05 Mol) Ammoniumchlorid in 350 ml Wasser zugegeben und eine halbe Stunde gerührt. Nach Einstellen des pH-Werts auf 8.9 - 9.0 durch Zugabe von wäßrigem Ammoniak wurde die Mischung weitere 3 Stunden lang bei 30 bis 35°C gerührt. Anschließend wurde das Reaktionsgemisch mit konz. Salzsäure angesäuert (pH 0.5 - 1.0) und mit Natriumbicarbonatlösung und Wasser nachgewaschen. Nach Abtrennung der wäßrigen Phase wurde das Methylenchlorid an einem Verdampfer abdestilliert. Es wurden 134 g (Reinheit lt. GC 84 %) Butyrylessigsäuremethylester als gelbliche Flüssigkeit erhalten (78 % Ausbeute).

### Beispiel 2:

56 g (1 Mol) Calciumoxid wurden in 650 ml Methylenchlorid vorgelegt und bei 20 bis 30°C wurden 116 g (1 Mol) Acetessigsäuremethylester innerhalb von 0.5 Stunden zugetropft. Anschließend wurde 1 Stunde lang bei dieser Temperatur nachgerührt. Nun wurden 116 g (1.05 Mol) Buttersäurechlorid innerhalb einer Stunde bei einer Temperatur von 30 - 35°C zudosiert und anschließend 3 Stunden lang bei dieser Temperatur nachgerührt. Zu der zähflüssigen Suspension wurden dann bei 30°C 54 g (1 Mol) Ammoniumchlorid in 300 ml Wasser gegeben und 30 Minuten lang gerührt. Der pH-Wert des Reaktionsgemisches wurde mit wäßrigem Ammoniak auf pH 9 eingestellt und 3 Stunden bei 30°C nachgerührt. Nach dem Ansäuern des Reaktionsgemisches mit konz. Salzsäure (pH <1) wurde mit Natriumbicarbonat-Lösung und Wasser nachgewaschen. Nach Abtrennung der Wasserphase wurde das Lösungsmittel am Verdampfer abdestilliert. Es wurden 141 g (Reinheit lt. GC 73 %) Butyrylessigsäuremethylester erhalten (Ausbeute 71 %).

### Beispiel 3:

Unter gleichen Bedingungen wie in Beispiel 1 wurden 116 g (1.0 Mol) Acetessigsäuremethylester in einem Gemisch aus 520 ml Methylenchlorid und 30 ml Methylethylketon mit 77.8 g (1.05 Mol) Calciumhydroxid und 106.5 g (1.15 Mol) Propionsäurechlorid umgesetzt. Der pH-Wert bei der Nachreaktion mit Ammoniak wurde auf 9.1 eingestellt. Erhalten wurden 117 g (Reinheit lt. GC 79.0 %) Propionylessigsäuremethylester (Ausbeute 71.0 %).

### Beispiel 4:

Unter gleichen Bedingungen wie in Beispiel 1 wurden 116 g (1.0 Mol) Acetessigsäuremethylester in einem Gemisch aus 520 ml Methylenchlorid und 30 ml Methylethylketon mit 77.8 g (1.05 Mol) Calciumhydroxid und 122.6 g (1.15 Mol) Isobuttersäurechlorid umgesetzt. Der pH-Wert bei der Nachreaktion mit Ammoniak wurde auf 9.2 eingestellt. Erhalten wurden 136 g (Reinheit lt. GC 79.8 %) Isobutyrylessigsäuremethylester (Ausbeute 75.4 %).

### Beispiel 5:

Unter gleichen Bedingungen wie in Beispiel 1 wurden 116 g (1.0 Mol) Acetessigsäuremethylester in einem Gemisch aus 520 ml Methylenchlorid und 30 ml Methylethylketon mit 77.8 g (1.05 Mol) Calciumhydroxid und 138.7 g (1.15 Mol) Valeriansäurechlorid umgesetzt. Der pH-Wert bei der Nachreaktion mit Ammoniak wurde auf 9.1 eingestellt. Erhalten wurden 151 g (Reinheit lt. GC 82.5 %) Valerylessigsäuremethylester (Ausbeute 79.0 %).

### Beispiel 6:

Unter gleichen Bedingungen wie in Beispiel 1 wurden 116 g (1.0 Mol) Acetessigsäuremethylester in einem Gemisch aus 520 ml Methylenchlorid und 30 ml Methylethylketon mit 77.8 g (1.05 Mol) Calciumhydroxid und 148.0 g (1.1 Mol) Hexansäurechlorid umgesetzt. Der pH-Wert bei der Nachreaktion mit Ammoniak wurde auf 9.2 eingestellt. Erhalten wurden 165 g (Reinheit lt. GC 78.5 %) Hexyrylessigsäuremethylester (Ausbeute 75.0 %).

### Beispiel 7:

Unter gleichen Bedingungen wie in Beispiel 1 wurden 116 g (1.0 Mol) Acetessigsäuremethylester in einem Gemisch aus 520 ml Methylenchlorid und 30 ml Methylethylketon mit 77.8 g (1.05 Mol) Calciumhydroxid und 176.7 g (1.1 Mol) Cyclohexylessigsäurechlorid umgesetzt. Die Nachreaktionszeit nach der Ammoniakdosierung dauerte bei einem pH-Wert von 9.5 6 Stunden lang. Erhalten wurden 196 g (Reinheit lt. GC 75.0 %) 4-Cyclohexylacetessigsäuremethylester (Ausbeute 74.5 %).

### Beispiel 8:

Unter gleichen Bedingungen wie in Beispiel 1 wurden 130 g (1.0 Mol) Acetessigsäureethylester in einem Gemisch aus 1.2 1 Methylenchlorid und 300 ml Methylethylketon mit 77.8 g (1.05 Mol) Calciumhydroxid und 106.5 g (1.15 Mol) Propionsäurechlorid umgesetzt. Die Nachreaktionszeit nach der Ammoniakdosierung dauerte bei einem pH-Wert von 9.3 4 Stunden lang. Erhalten wurden 134 g (Reinheit lt. GG 72.0 %) Propionylessigsäureethylester (Ausbeute 67.0 %).

Nach fraktionierter Destillation konnten die beispielsgemäßen β-Ketocarbonsäureester in einer Reinheit von 96 bis 99 % erhalten werden.

## Patentansprüche

1. Verfahren zur Herstellung von β-Ketocarbonsäureestern der allgemeinen Formel 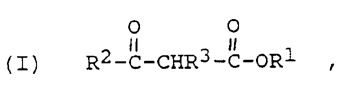 wobei R¹ für einen Alkylrest mit 1 bis 4 C-Atomen steht, R² einen Alkylrest oder Alkenylrest mit 2 bis 15 C-Atomen oder einen Phenylrest bedeutet und R³ Wasserstoff oder einen Alkyl- oder Alkenyl-Rest mit 1 bis 6 C-Atomen bedeutet, in welchem Acetcarbonsäure ester der allgemeinen Formel 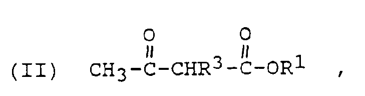 wobei R¹ und R³ die angegebenen Bedeutungen haben, mit Calciumhydroxid oder Calciumoxid in Gegenwart eines organischen Lösungsmittels und unter Ausschluß von Wasser umgesetzt werden, die gebildeten Calcium-Chelatkomplexe mit Carbonsäurechlorid acyliert werden, dadurch gekennzeichnet, daß die acylierten Calcium-Chelatkomplexe anschließend mit wäßriger Ammoniumsalzlösung unter Bildung der β-Ketocarbonsäureester der Formel (I) gespalten werden.

2. Verfahren nach Anspruch 1, dadurch gekennzeichnet, daß als Acetcarbonsäureester Acetessigsäureester eingesetzt werden.

3. Verfahren nach Anspruch 1 oder 2, dadurch gekennzeichnet, daß zur Darstellung des Calcium-Chelatkomplexes Calciumhydroxid eingesetzt wird.

4. Verfahren nach Anspruch 1, 2 oder 3, dadurch gekennzeichnet, daß die Calciumverbindung in mindestens äquivalenter Menge zum Acetcarbonsäureester eingesetzt wird.

5. Verfahren nach Anspruch 1, 2, 3 oder 4, dadurch gekennzeichnet, daß aprotische Lösungsmittel eingesetzt werden.

6. Verfahren nach Anspruch 1, 2, 3, 4 oder 5, dadurch gekennzeichnet, daß als Lösungsmittel Dichlormethan oder Gemische mit Dichlormethan eingesetzt werden.

7. Verfahren nach Anspruch 1, 2, 3, 4, 5 oder 6, dadurch gekennzeichnet, daß das Carbonsäurechlorid in mindestens äquivalenter Menge, bezogen auf den Acetcarbonsäureester, der Suspension zugegeben wird.

8. Verfahren nach Anspruch 1, 2, 3, 4, 5, 6 oder 7, dadurch gekennzeichnet, daß die Spaltung des acylierten Calcium-Chelatkomplexes in wäßriger Ammoniumchloridlösung bei einem pH-Wert von 8.8 bis 9.5 erfolgt.

## Claims

1. A process for the preparation of β-ketocarboxylic acid esters of the general formula 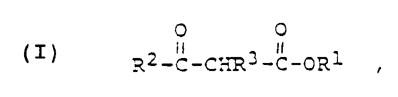 in which R¹ is an alkyl radical having 1 to 4 C atoms, R² is an alkyl radical or alkenyl radical having 2 to 15 C atoms or a phenyl radical and R³ is hydrogen or an alkyl or alkenyl radical having 1 to 6 C atoms, in which acetocarboxylic acid esters of the general formula 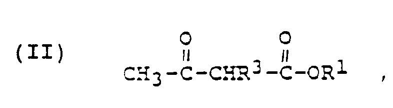 in which R¹ and R³ are as defined, are reacted with calcium hydroxide or calcium oxide in the presence of an organic solvent and in the absence of water, the calcium chelate complexes formed are acylated with carboxylic acid chloride, characterised in that the acylated calcium chelate complexes are then cleaved with aqueous ammonium salt solution to form the β-ketocarboxylic acid esters of formula (I).

2. A process according to Claim 1, characterised in that acetoacetic acid esters are used as the acetocarboxylic acid esters.

3. A process according to Claim 1 or 2, characterised in that calcium hydroxide is used to prepare the calcium chelate complex.

4. A process according to Claim 1, 2 or 3, characterised in that the calcium compound is used in at least an equivalent amount based on the acetocarboxylic acid ester.

5. A process according to Claim 1, 2, 3 or 4, characterised in that aprotic solvents are used.

6. A process according to Claim 1, 2, 3, 4 or 5, characterised in that methylene chloride or mixtures with methylene chloride are used as the solvents.

7. A process according to Claim 1, 2, 3, 4, 5 or 6, characterised in that the carboxylic acid chloride is added to the suspension in at least an equivalent amount based on the acetocarboxylic acid ester.

8. A process according to Claim 1, 2, 3, 4, 5, 6 or 7, characterised in that the cleavage of the acylated calcium chelate complex takes place in aqueous ammonium chloride solution at a pH of 8.8 to 9.5.

## Revendications

1. Procédé de préparation d'esters d'acides β-cétocarboxyliques de formule générale 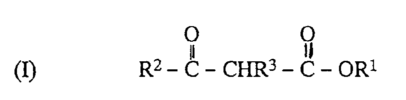 dans laquelle R¹ représente un reste alkyle de 1 à 4 atomes de carbone, R² représente un reste alkyle ou un reste alcényle de 2 à 15 atomes de carbone ou un reste phényle, et R³ représente l'hydrogène ou un reste alkyle ou alcényle de 1 à 6 atomes de carbone, dans lequel on fait réagir un ester d'acide acétocarboxylique de formule générale 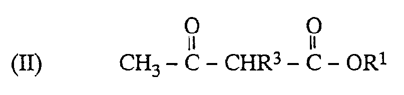 dans laquelle R¹ et R³ ont les significations indiquées, avec de l'hydroxyde de calcium ou de l'oxyde de calcium en présence d'un solvant organique et dans des conditions anhydres, on acyle les complexes de type chélate de calcium formés avec un chlorure d'acide carboxylique, caractérisé en ce que l'on dissocie ensuite les complexes de type chélate de calcium acylés avec une solution de sel d'ammonium en formant les β-cétoesters de formule (I).

2. Procédé selon la revendication 1, caractérisé en ce que, comme ester d'acide acétocarboxylique, on utilise un ester de l'acide acétoacétique.

3. Procédé selon la revendication 1 ou 2, caractérisé en ce que l'on utilise de l'hydroxyde de calcium pour former le complexe de type chélate de calcium.

4. Procédé selon la revendication 1, 2 ou 3, caractérisé en ce que l'on introduit le composé de calcium en une quantité au moins équivalente à l'ester d'acide acétocarboxylique.

5. Procédé selon la revendication 1, 2, 3 ou 4, caractérisé en ce que l'on utilise des solvants aprotiques.

6. Procédé selon la revendication 1, 2, 3, 4 ou 5, caractérisé en ce que l'on utilise comme solvant du dichlorométhane ou des mélanges contenant du dichlorométhane.

7. Procédé selon la revendication 1, 2, 3, 4, 5 ou 6, caractérisé en ce que l'on ajoute le chlorure d'acide carboxylique à la suspension en une quantité au moins équivalente par rapport à l'ester d'acide acétocarboxylique.

8. Procédé selon la revendication 1, 2, 3, 4, 5, 6 ou 7, caractérisé en ce que la dissociation du complexe de type chélate de calcium acylé s'effectue dans une solution aqueuse de chlorure d'ammonium à un pH de 8,8 à 9,5.
